# EUROPEAN PATENT SPECIFICATION

(11) **EP 0 937 131 B1**
(45) Date of publication and mention of the grant of the patent: **10.01.2001**
(21) Application number: 97943054.3
(22) Date of filing: 26.09.1997
(51) Int. Cl.: C11D 9/28, C11D 3/00, C11D 3/48, C11D 17/00

(54) **ANTISEPTIC SOAP BAR**
ANTISEPTISCHER SEIFENRIEGEL
PAIN DE SAVON ANTISEPTIQUE

(30) Priority: 09.11.1996 GB 9623377
(43) Date of publication of application: 25.08.1999
(73) Proprietor: RECKITT & COLMAN PRODUCTS LIMITED, London W4 2RW (GB)
(72) Inventor: PAYNE, David, Norman, Hull, HU10 7QL (GB)
(74) Representative: Dickson, Elizabeth Anne
(86) International application number: GB9702648
(87) International publication number: WO9821306

(56) References cited:
- EP-A- 0 029 805

## Description

This invention relates to soap bars having good antiseptic activity and in particular to soap bars containing reduced concentrations of chelating agents.

Many so-called medicated soap products are available through the world, the majority still being sold in the form of bar soaps. Such products are generally referred to as antiseptic if they contain one or more antimicrobial agents, although their actual efficacy in use is rarely substantiated.

Thus many products contain higher than necessary levels of antimicrobial agents (resulting in increased costs and wastage) or levels that are not actually effective in use.

It is well known that the activity of many antimicrobial agents may be improved by the use of chelating agents and such agents are often used indiscriminately in such products as antiseptic soaps.

In fact chelating agents are often used as stabilising agents in the soap base but are then added again during manufacture of the finished soap, in the belief that they will increase antimicrobial activity.

However, there are a number of drawbacks associated with the use of chelating agents, notably their cost, their potential as irritants and their environmental impact (eg their effect on river life etc).

Thus there is a continuing need for soap bar products having good, measurable, antiseptic activity but using the lowest possible concentrations of antimicrobial agents and chelating agents.

We have now found that a particular combination of active agents will produce satisfactory antiseptic activity in a bar soap (including a substantive effect on the skin) without the use of high levels of EDTA chelating agent.

There is therefore provided an antiseptic bar soap comprising:
a) 0.35 to 0.75% w/w of an antimicrobially active chlorinated methyl substituted phenol;
b) 0.15 to 0.25% w/w triclosan (or a salt thereof); and
c) less than 0.075% w/w (preferably less than 0.05% w/w) EDTA chelating agent.

Preferably the antiseptic bar soaps of the present invention contain no chelating agents other than those included in the soap base as stabilising agents.

Chelating agents that may be included as stabilising agents in the soap base of the antiseptic bar soaps of the invention include EDTA (ethylene diamine tetraacetic acid and its soluble salts). Preferably the antiseptic bar soaps of the invention comprise less than 0.05% w/w EDTA and most preferably about 0.025% w/w EDTA.

The antimicrobially active chlorinated methyl substituted phenol used in the antiseptic bar soaps of the invention are preferably compounds of formula I 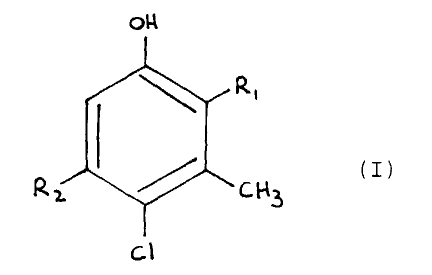 wherein
- R₁: is chlorine or hydrogen, and
- R₂: is methyl or hydrogen

Preferred compounds of formula I include PCMC (4-chloro-3-methylphenol), PCMX (4-chloro-3,5-dimethylphenol), DCMX (2,4-dichloro-3,5-dimethylphenol) or mixtures thereof.

Preferably the antiseptic bar soaps of the invention comprise from 0.4 to 0.6% w/w of a compound of formula I, more preferably about 0.5% w/w.

Preferably the antiseptic bar soaps of the invention comprise about 0.2% w/w triclosan (2-hydroxy-4,2', 4'-trichloro-diphenyl ether) or a salt thereof.

The antiseptic bar soaps of the invention also comprise a conventional soap base, which will generally comprise saponified fatty acids derived from natural sources. Examples of suitable soap bases include an 80:20 mixture of tallow and coconut oil fatty acids and an 80:20 mixture of distilled palm oil fatty acids and distilled palm kernel fatty acids. As stated previously the soap base may further comprise chelating agents as stabilisers therefore but it will preferably comprise no more than 0.05% w/w total chelating agents and most preferably no more than 0.025% w/w EDTA.

The antiseptic bar soaps of the invention may contain further conventional additives eg. colours, fragrances (eg pine oil), etc.

The antiseptic bar soaps of the invention may be produced by any conventional method, such as mixing of the components together in a hopper, roll milling, extruding and then cutting and stamping into the final shape.

The invention will be illustrated by the following Examples.

### Example 1

An antiseptic soap bar is produced to the following formula:

| | % w/w |
|---|---|
| PCMX | 0.5 |
| Triclosan | 0.2 |
| Pine Oil (fragrance) | 1.5 |
| Colours * | 0.12 |
| Water * | 0.13 |
| DFA Soap | to 100% |
| Chips | |

| | |
|---|---|
| DFA Soap Chips: Distilled fatty acids of Palm Oil 80 +/-3% Distilled fatty acids of palm kernel oil 20 +/- 3% Additions: EDTA tetra sodium 0.025%. Also 0.2% EHDP which is [phosphoric acid (1-hydroxy-ethylidene) bis] is added as a stabiliser. * The colours and water are premixed and closed as a colour solution. | |

The components are mixed together in a hopper and these then go through a roll mill,at ambient temperature and pressure. From there the mixture is passed into a plodder (extruder) also at ambient temperature and then from the extruder it is chopped and stamped into the requisite shape.

The soap bar has good antiseptic activity and is substantive on the skin.

### Example 2

The substantive activity of soap bars of the invention was demonstrated in the following test.

Standard soap solutions were made by dissolving a tallow soap base in standard hard water to give a concentration of 8% (approximately the same concentration as in lather during hand washing). Three solutions were prepared each containing 0.04% w/w PCMX (equivalent to 0.5% w/w in a soap bar) and either 0.008, 0.016, or 0.024% w/w triclosan (equivalent to 0.1, 0.2 and 0.3% w/w in a soap bar).

Volunteers dabbed a finger onto the centre of a Tryptone Soya Agar plate for 30 seconds, washed their hands for 1 minute in 5ml of one of the above solutions, rinsed them, and again dabbed their finger onto an agar plate.

The agar plates were then swabbed with a suspension of either *Staphylococcus aureus* or *Escherichia coil* and incubated overnight at 30°C. They were inspected next day for the presence of a zone of inhibition.

In all cases the unwashed hands, and those washed in solutions equivalent to 0.1% w/w triclosan containing soaps, produced no zones of inhibition; indicating no substantive effect on the skin.

In all cases the hands washed in solutions equivalent to 0.2% or 0.3% w/w triclosan containing soaps produced distinct zones of inhibition; indicating a substantive antimicrobial effect on the skin.

This indicates that more than 0.1% w/w triclosan is necessary to produce a soap bar with substantive antiseptic activity, but that more than 0.2% w/w triclosan is not necessary (even in the absence of added EDTA).

## Claims

1. An antiseptic bar soap, the bar soap comprising:
a) 0.35 to 0.75% w/w of an antimicrobially active chlorinated methyl substituted phenol;
b) 0.15 to 0.25% w/w triclosan (or a salt thereof); and
c) less than 0.075% w/w EDTA chelating agent.

2. An antiseptic bar soap according to Claim 1 in which any chelating agents present are in the soap base of the bar soap as stabilising agents.

3. An antiseptic bar soap according to Claim 1 or Claim 2 in which the chelating agents included as stabilising agents in the soap base are selected from ethylene diamine tetraacetic acid (EDTA) and its soluble salts and [phosphoric acid (1-hydroxy ethylidene) bis].

4. An antiseptic bar soap according to any one of the preceding claims comprising less than 0.05% w/w EDTA, preferably about 0.025% w/w EDTA.

5. An antiseptic bar soap according to any one of the preceding claims in which the antimicrobially active chlorinated methyl substituted phenol used in the antiseptic bar soaps of the invention are preferably compounds of Formula I 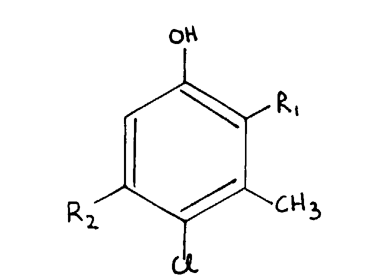 wherein
R₁ is chlorine or hydrogen, and
R₂ is methyl or hydrogen

6. An antiseptic bar soap according to Claim 5 in which the compounds of formula I are selected from 4-chloro-3-methylphenol, 4-chloro-3,5-dimethylphenol, 2,4-dichloro-3,5-dimethylphenol and mixtures thereof.

7. An antiseptic bar soap according to Claim 5 or Claim 6 comprise from 0.4 to 0.6% w/w of a compound of formula I, defined in Claim 5.

8. An antiseptic bar soap according to Claim 5 or Claim 6 comprise about 0.5% w/w of a compound of formula I, defined in Claim 5.

9. An antiseptic bar soap according to any one of the preceding claims which comprise about 0.2% w/w triclosan (2-hydroxy-4,2¹, 4¹-trichloro-diphenyl ether) or a salt thereof.

10. An antiseptic bar soap as claimed in any one of the preceding claims in which the soap base comprises saponified fatty acids derived from natural sources, preferably an 80:20 mixture of tallow and coconut oil fatty acids and an 80:20 mixture of distilled palm oil fatty acids and distilled palm kernel fatty acids

## Patentansprüche

1. Antiseptische feste Seife, die die folgenden Bestandteile umfasst:
a) 0,35 bis 0,75 % g/g eines antimikrobiell wirksamen, chlorierten, methylsubstituierten Phenols;
b) 0,15 bis 0,25 % g/g Triclosan (oder eines Salzes hiervon); und
c) weniger als 0,075 % g/g EDTA-Chelatbildner.

2. Antiseptische feste Seife nach Anspruch 1, wobei jegliche vorhandenen Chelatbildner in der Seifengrundlage der festen Seife als Stabilisierungsmittel vorliegen.

3. Antiseptische feste Seife nach Anspruch 1 oder Anspruch 2, wobei die in der Seifengrundlage als Stabilisierungsmittel vorhandenen Chelatbildner aus Ethylendiamintetraessigsäure (EDTA) und den löslichen Salzen hiervon und (1-Hydroxyethyliden)bis-phosphorsäure ausgewählt sind.

4. Antiseptische feste Seife nach einem der vorhergehenden Ansprüche, die weniger als 0,05 % g/g EDTA, vorzugsweise etwa 0,025 % g/g EDTA umfaßt.

5. Antiseptische feste Seife nach einem der vorhergehenden Ansprüche, wobei es sich bei dem in den erfindungsgemäßen antiseptischen festen Seifen verwendeten, antimikrobiell wirksamen, chlorierten methylsubstituierten Phenol vorzugsweise um Verbindungen der folgenden Formel I handelt: 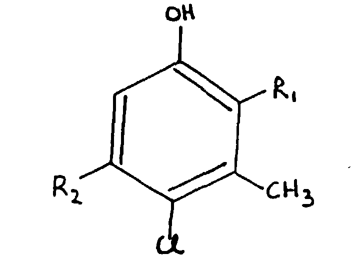 worin R₁ für Chlor oder Wasserstoff steht und R₂ für Methyl oder Wasserstoff steht.

6. Antiseptische feste Seife nach Anspruch 5, wobei die Verbindungen der Formel I aus 4-Chlor-3-methylphenol, 4-Chlor-3,5-dimethylphenol, 2,4-Dichlor-3,5-dimethylphenol und Gemischen hiervon ausgewählt sind.

7. Antiseptische feste Seife nach Anspruch 5 oder Anspruch 6, die 0,4 bis 0,6 % g/g einer Verbindung der Formel I gemäß Definition in Anspruch 5 umfaßt.

8. Antiseptische feste Seife nach Anspruch 5 oder Anspruch 6, die etwa 0,5 % g/g einer Verbindung der Formel I gemäß Definition in Anspruch 5 umfaßt.

9. Antiseptische feste Seife nach einem der vorhergehenden Ansprüche, die etwa 0,2 % g/g Triclosan (2-Hydroxy-4,2¹,4¹-trichlor-diphenylether) oder eines Salzes hiervon umfaßt.

10. Antiseptische feste Seife nach einem der vorhergehenden Ansprüche, worin die Seifengrundlage aus natürlichen Quellen herrührende, verseifte Fettsäuren, vorzugsweise ein 80:20-Gemisch aus Talg- und Kokosölfettsäuren und ein 80:20-Gemisch aus destillierten Palmöl- und destillierten Palmkernölfettsäuren, umfaßt.

## Revendications

1. Pain de savon antiseptique, ledit pain de savon comprenant :
a) de 0,35 à 0,75 % en poids/poids d'un phénol chloré à substitution méthyle ayant une activité antimicrobienne ;
b) de 0,15 à 0,25 % en poids/poids de triclosan (ou d'un sel de celui-ci) ; et
c) moins de 0,075 % en poids/poids d'agent chélateur EDTA.

2. Pain de savon antiseptique selon la revendication 1, dans lequel tous les agents chélateurs présents se trouvent dans la base de savon du pain de savon en tant qu'agents stabilisants.

3. Pain de savon antiseptique selon la revendication 1 ou la revendication 2, dans lequel les agents chélateurs inclus en tant qu'agents stabilisants dans la base de savon sont choisis parmi l'acide éthylènediamine-tétra-acétique (EDTA) et ses sels solubles et [acide phosphorique (1-hydroxyéthylidène)bis].

4. Pain de savon antiseptique selon l'une quelconque des revendications précédentes, comprenant moins de 0,05 % en poids/poids de EDTA, de préférence environ 0,025 % en poids/poids de EDTA.

5. Pain de savon antiseptique selon l'une quelconque des revendications précédentes, dans lequel le phénol chloré à substitution méthyle ayant une activité antimicrobienne qui est utilisé dans les pains de savon antiseptiques de l'invention est constitué de préférence par des composés de formule I 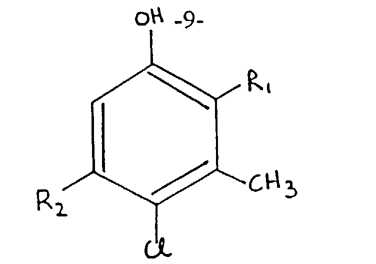 dans laquelle R₁ est le chlore ou l'hydrogène et R₂ est le groupe méthyle ou l'hydrogène.

6. Pain de savon antiseptique selon la revendication 5, dans lequel les composés de formule I sont choisis parmi les 4-chloro-3-méthylphénol, 4-chloro-3,5-diméthylphénol, 2,4-dichloro-3,5-diméthylphénol et des mélanges de ceux-ci.

7. Pain de savon antiseptique selon la revendication 5 ou la revendication 6, comprenant de 0,4 à 0,6 % en poids/poids d'un composé de formule I tel que défini dans la revendication 5.

8. Pain de savon antiseptique selon la revendication 5 ou la revendication 6, comprenant environ 0,5 % en poids/poids d'un composé de formule I tel que défini dans la revendication 5.

9. Pain de savon antiseptique selon l'une quelconque des revendications précédentes, comprenant environ 0,2 % en poids/poids de triclosan (éther 2-hydroxy-4,2¹,4¹-trichlorodiphénylique) ou d'un sel de celui-ci.

10. Pain de savon antiseptique selon l'une quelconque des revendications précédentes, dans lequel la base du pain de savon comprend des acides gras saponifiés dérivés de sources naturelles, de préférence un mélange (80:20) d'acides gras de suif et d'huile de coco et un mélange (80:20) d'acides gras distillés d'huile de palme et d'acides gras distillés de palmiste
